(19) Europäisches Patentamt
European Patent Office
Office européen des brevets

(11) Numéro de publication : **0 484 204 A1**

(12) **DEMANDE DE BREVET EUROPEEN**

(21) Numéro de dépôt : **91402825.3**

(22) Date de dépôt : **23.10.91**

(51) Int. Cl.[5] : **C07D 487/04**, C07D 471/14, A61K 31/55, // (C07D487/04, 243:00, 235:00), (C07D471/14, 243:00, 221:00)

(30) Priorité : **24.10.90 GB 9023155**

(43) Date de publication de la demande : **06.05.92 Bulletin 92/19**

(84) Etats contractants désignés : **AT BE CH DE DK ES FR GB GR IT LI LU NL SE**

(71) Demandeur : **ROUSSEL-UCLAF**
**35, Boulevard des Invalides**
**F-75007 Paris (FR)**

(72) Inventeur : **Hedgecock, Charles John Robert**
**19 Longleaze, Wootton Basset**
**Swindon, Wiltshire SN4 8AX (GB)**
Inventeur : **Jones, Stuart Donald**
**1 Walnut Tree Gardens, Lydiard Millicent**
**Swindon, Wiltshire SN5 9LH (GB)**

(74) Mandataire : **Vieillefosse, Jean-Claude et al**
**Roussel-Uclaf 111, route de Noisy B.P. 9**
**F-93230 Romainville (FR)**

(54) **7-Cycloalkylimidazodiazépines et leurs sels, procédé de préparation et produits intermédiaires obtenus, application à titrede médicaments et compositions pharmaceutiques les renfermant.**

(57) L'invention concerne les produits (I) :

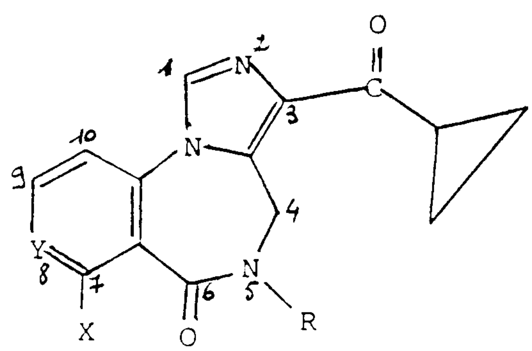

5I)

dans laquelle
Y représente un azote, un carbone lié à Z, Z représentant hydrogène, halogène, $-C{\equiv}N$, $-N_3$, $-C(Hal)_3$ ; X représente un cycloalkyle ($C_{3-6}$) ou un hydrogène étant entendu que lorsque X est hydrogène, Y est azote,
R représente hydrogène, alkyle ($C_{1-5}$), phényle, phényle substitué, ainsi que leurs sels, leur procédé et les intermédiaires de préparation et leur application comme médicaments.

EP 0 484 204 A1

La présente invention concerne de nouvelles 7-cycloalkyl-imidazodiazépines et leurs sels, ainsi que le procédé de préparation et les produits intermédiaires obtenus, l'application à titre de médicaments de ces nouveaux produits et les compositions pharmaceutiques les renfermant.

L'invention a pour objet de nouvelles 7-cycloalkylimidazodiazépines répondant à la formule générale (I) :

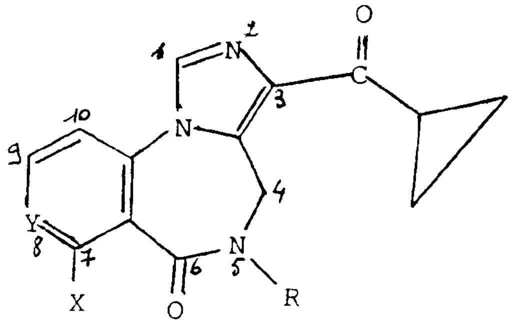

(I)

dans laquelle :

– Y représente un atome d'azote, un atome de carbone lié à un substituant Z dans lequel Z représente un atome d'hydrogène, un atome d'halogène, un groupement nitrile, un groupement

– $N_3$, un groupement -C(Hal)$_3$ dans lequel Hal représente un atome d'halogène,

– X représente un groupement cycloalkyle renfermant de 3 à 6 atomes de carbone ou un atome d'hydrogène étant entendu que lorsque X représente un atome d'hydrogène, Y représente un atome d'azote,

– R représente un atome d'hydrogène, un radical alkyle renfermant de 1 à 5 atomes de carbone, un groupement phényle, un groupement phényle substitué, ainsi que leurs sels d'addition avec les acides minéraux ou organiques.

Dans la formule générale (I) et dans ce qui suit :

– par atome d'halogène, on entend de préférence un atome de fluor, de chlore ou de brome ;

– par groupement cycloalkyle renfermant de 3 à 6 atomes de carbone, on entend un radical cyclopropyle, cyclobutyle, cyclopentyle ou cyclohexyle.

– par radical alkyle renfermant de 1 à 5 atomes de carbone, on entend un radical méthyle, éthyle, propyle, isopropyle, butyle, isobutyle, tertbutyle ou pentyle linéaire ou ramifié ;

– par groupement phényle substitué on entend de préférence un groupement phényle substitué par un atome d'halogène, un radical alkyle renfermant de 1 à 3 atomes de carbone, un radical alkoxy renfermant de 1 à 3 atomes de carbone, un groupement trifluorométhyle ;

– par radical alkyle renfermant de 1 à 3 atomes de carbone, on entend un radical méthyle, éthyle, propyle ou isopropyle ;

– par radical alkoxy renfermant de 1 à 3 atomes de carbone, on entend un radical méthoxy, éthoxy, propoxy ou isopropoxy.

Les sels d'addition avec les acides minéraux ou organiques peuvent être, par exemple, les sels formés avec les acides chlorhydrique, bromhydrique, iodhydrique, nitrique, sulfurique, phosphorique, propionique, formique, benzoïque, maléïque, fumarique, succinique, tartrique, citrique, oxalique, glyoxylique, aspartique, alcanesulfoniques, tel que l'acide méthanesulfonique et arylsulfoniques tel que l'acide benzènesulfonique.

Parmi les produits, objet de l'invention, on peut citer notamment les dérivés répondant à la formule (I) ci-dessus, caractérisés en ce que dans ladite formule (I), Y représente un atome d'azote ou un atome de carbone lié à un substituant Z dans lequel Z représente un atome d'hydrogène, X et R ont la signification déjà indiquée, ainsi que leurs sels d'addition avec les acides minéraux ou organiques.

Parmi ceux-ci, on peut citer les dérivés répondant à la formule (I) ci-dessus, caractérisés en ce que dans ladite formule (I), X représente un groupement cyclopropyle, Y et R ont la signification déjà indiquée, ainsi que leurs sels d'addition avec les acides minéraux ou organiques.

Parmi ces derniers on retient tout particulièrement les dérivés de formule (I) dont les noms suivent :

– La 7-cyclopropyl-5,6-dihydro-5-méthyl-6-oxo 4H-imidazo [1,5-a] [1,4] benzodiazépin-3-yl cyclopropyl méthanone ainsi que ses sels d'addition avec les acides minéraux ou organiques.

– La 7-cyclopropyl-5,6-dihydro-5-méthyl-6-oxo 4H-imidazo [1,5-a] pyrido [3,4-f] diazépin-3-yl cyclopropyl méthanone ainsi que ses sels d'addition avec les acides minéraux ou organiques.

L'invention a également pour objet un procédé de préparation des nouvelles 7-Cycloalkyl-imidazodiazépines telles que définies par la formule (I) ci-dessus, ainsi que de leurs sels, caractérisé en ce que :

a) pour préparer des produits de formule (I) dans laquelle Y représente un atome d'azote,

soit l'on soumet un composé de formule (II) :

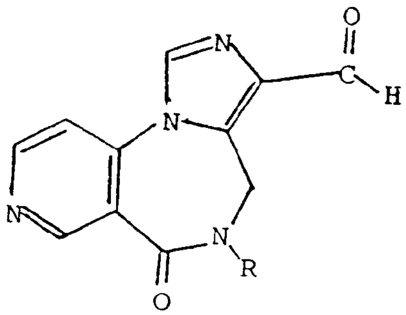

(II)

dans laquelle R a la signification déjà indiquée à l'action d'un dérivé magnésien d'un composé de formule (III) :

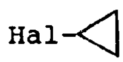

(III)

dans laquelle Hal représente un atome d'halogène pour obtenir un composé de formule (IV) :

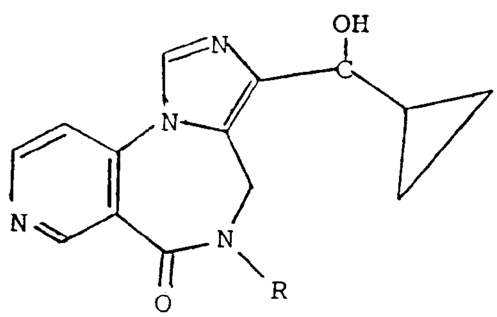

(IV)

dans laquelle R a la signification déjà indiquée, que l'on soumet à l'action d'oxydation pour obtenir le composé de formule (I) correspondant dans laquells X représente un atome d'hydrogène répondant à la formule ($I_A$) :

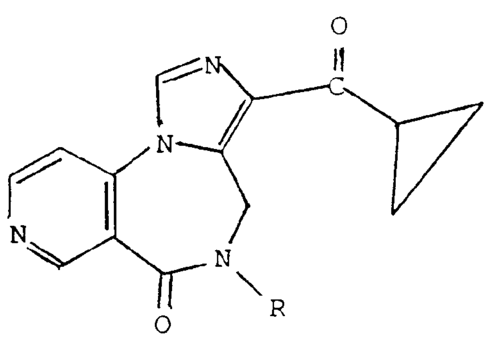

($I_A$)

dans laquelle R a la signification indiquée précédemment, que l'on peut salifier, si désiré,
soit l'on soumet un produit de formule (V) :

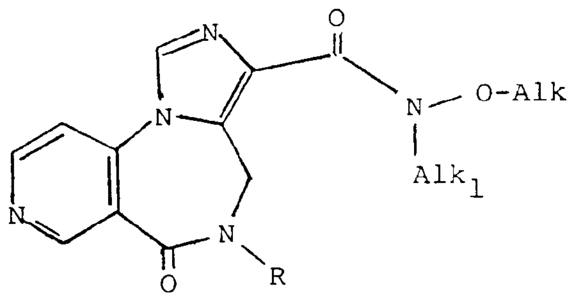

(V)

dans laquelle R a la signification indiquée précédemment et alk et $alk_1$ identiques ou différents représentent un radical alkyle renfermant de 1 à 3 atomes de carbone, à l'action d'un dérivé magnésien d'un composé de formule (III) :

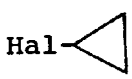

(III)

tel que défini ci-dessus
pour obtenir un produit de formule ($I_A$) tel que défini ci-dessus, que l'on peut salifier si désiré,
produit de formule ($I_A$) que l'on soumet le cas échéant à l'action d'un dérivé magnésien d'un composé de formule (VI) :

Hal-X' (VI)

dans laquelle Hal représente un atome d'halogène et X' représente un radical cycloalkyle renfermant de 3 à 6 atomes de carbone pour obtenir un composé de formule (VII) :

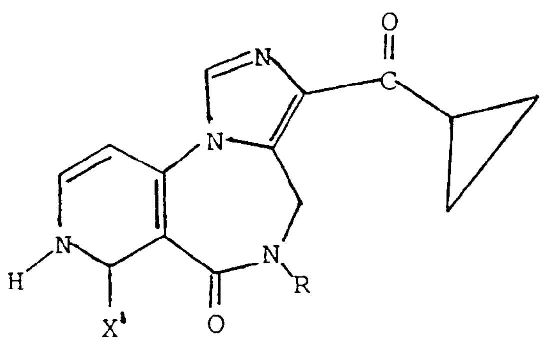

(VII)

dans laquelle X' et R ont la signification indiquée précédemment que l'on soumet à l'action d'un agent d'oxydation pour obtenir le composé de formule (I) correspondant dans laquelle X représente un radical cycloalkyle renfermant de 3 à 6 atomes de carbone répondant à la formule ($I_B$) :

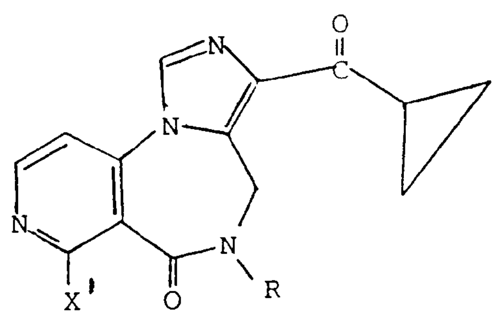

($I_B$)

dans laquelle X' et R ont la signification indiquée précédemment, que l'on peut salifier si désiré,
b) pour préparer des produits de formule (I) dans laquelle Y représente un atome de carbone lié à un substituant Z tel que défini précédemment,
soit l'on soumet un composé de formule (VIII) :

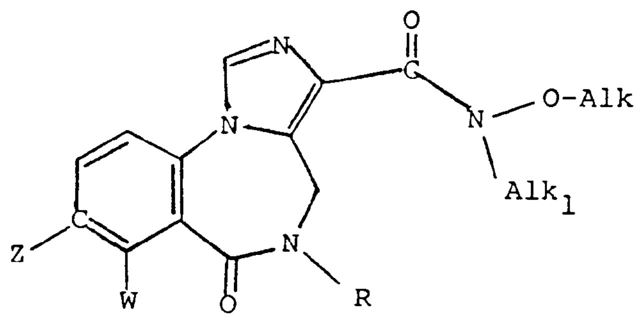

(VIII)

dans laquelle R, Z, Alk et $Alk_1$ ont la signification indiquée précédemment et W représente un atome de fluor ou de chlore, à l'action d'un dérivé magnésien d'un composé de formule (III) :

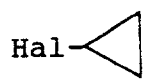

et tel que défini précédemment pour obtenir un composé de formule (IX) :

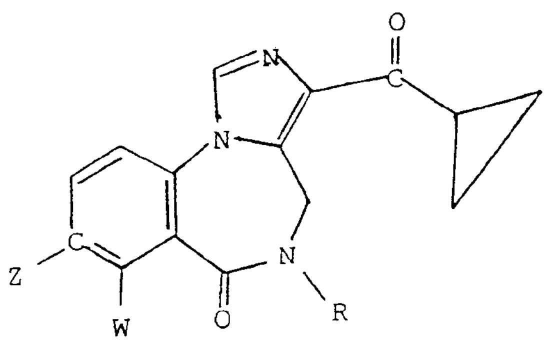

(IX)

dans laquelle Z, W et R ont la signification indiquée précédemment,
soit l'on soumet un composé de formule (X) :

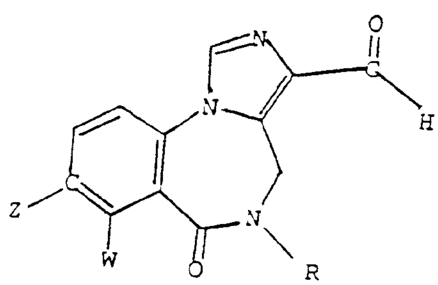

(X)

dans laquelle Z, W et R ont la signification indiquée précédemment, à l'action d'un dérivé magnésien d'un composé de formule (III) :

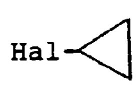

(III)

tel que défini ci-dessus pour obtenir un composé de formule (XI) :

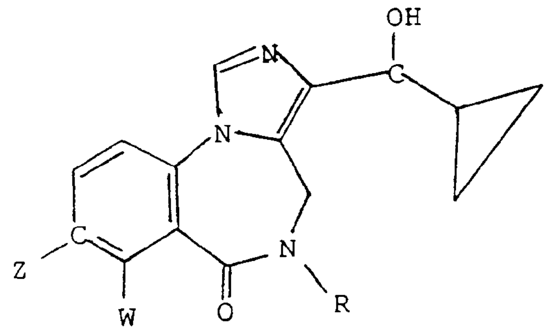

(XI)

dans laquelle Z, W et R ont la signification indiquée précédemment, que l'on soumet à l'action d'un agent d'oxydation pour obtenir un composé de formule (IX) tel que défini précédemment, composé de formule (IX) que l'on soumet à l'action d'un dérivé magnésien d'un composé de formule (VI) :

Hal-X' (VI)

tel que défini ci-dessus pour obtenir le composé de formule (I) correspondant dans laquelle X représente un radical cycloalkyle répondant à la formule ($I_C$) :

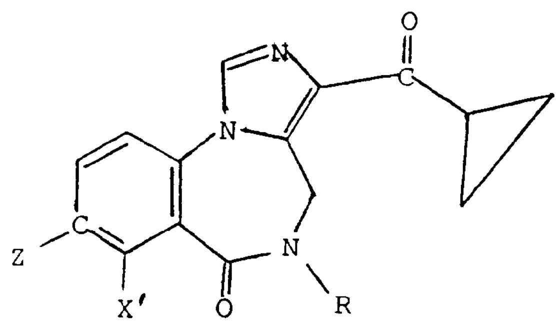

($I_C$)

dans laquelle Z, X' et R ont la signification indiquée précédemment que l'on peut salifier, si désiré.

Dans des conditions préférentielles de mise en oeuvre de l'invention, le procédé de préparation ci-dessus décrit est caractérisé en ce que :

– la réaction du produit de formule (II), (V), (VIII) ou (X) avec le dérivé magnésien d'un produit de formule (III) ou la réaction du produit de formule ($I_A$) ou (IX) avec le dérivé magnésien d'un produit de formule (VI) est effectuée au sein d'un solvant organique anhydre tel que le tétrahydrofuranne,

– l'oxydation du produit de formule (IV), (VII) ou (XI) est réalisée au moyen d'un oxydant doux tel que le dioxyde de manganèse, au sein d'un solvant organique anhydre tel que le dichlorométhane.

Dans le composé de formule (VIII) ou (X), W représente avantageusement un atome de fluor.

– Les composés de formule ($I_B$) ou ($I_C$) dans laquelle X' représente un radical cyclopropyle sont préparés en traitant respectivement le composé de formule (V) ou (VIII) telle que définie ci-dessus avec un excès du dérivé magnésien du composé de formule (III) tel que défini ci-dessus sans isoler intermédiairement les composés de formule ($I_A$) ou (IX) respectivement.

Les produits de formule (I) présentent un caractère basique. On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques, on note en particulier des propriétés agonistes inverses des benzodiazépines.

Certains produits présentent, en outre, des propriétés tranquilisantes.

Ces propriétés sont illustrées plus loin dans la partie expérimentale.

Ces propriétés justifient l'utilisation des nouvelles 7-Cycloalkyl-imidazodiazépines répondant à la formule (I) ainsi que de leurs sels d'addition avec les acides pharmaceutiquement acceptables, à titre de médicaments.

La présente invention a aussi également pour objet l'application à titre de médicaments des nouvelles 7-Cycloalkyl-imidazodiazépines telles que définies par la formule générale (I) ainsi que de leurs sels d'addition avec les acides minéraux ou organiques pharmaceutiquement acceptables.

Parmi les médicaments, objet de l'invention, on retient notamment les médicaments caractérisés en ce qu'ils sont constitués par les nouvelles 7-Cycloalkyl-imidazodiazépines répondant à la formule (I) dans laquelle

Y représente un atome d'azote ou un atome de carbone lié à un substituant Z dans lequel Z représente un atome d'hydrogène, X et R ont la signification déjà indiquée, ainsi que leurs sels d'addition avec les acides minéraux ou organiques pharmaceutiquement acceptables.

Parmi ceux-ci, on retient tout particulièrement ceux répondant à la formule (I) ci-dessus dans laquelle X représente un groupement cyclopropyle, Y et R ont la signification déjà indiquée, ainsi que leurs sels d'addition avec les acides minéraux ou organiques pharmaceutiquement acceptables.

Parmi les médicaments préférés de l'invention, on retient tout particulièrement :

– La 7-cyclopropyl-5,6-dihydro-5-méthyl-6-oxo 4H-imidazo [1,5-a] [1,4] benzodiazépin-3-yl cyclopropyl méthanone ainsi que ses sels d'addition avec les acides minéraux ou organiques pharmaceutiquement acceptables,

– la 7-cyclopropyl-5,6-dihydro-5-méthyl-6-oxo 4H-imidazo [1,5-a] pyrido [3,4-f] diazépin-3-yl cyclopropyl méthanone ainsi que ses sels d'addition avec les acides minéraux ou organiques pharmaceutiquement acceptables.

Ces médicaments trouvent, par exemple, leur emploi dans le traitement des troubles de la mémoire, notamment en gériatrie, ainsi que dans le traitement de la sénescence cérébrale. Certains produits peuvent également être utilisés dans le traitement de l'obésité ainsi que comme tranquilisant mineur dans le traitement de certaines agitations ou irritabilité et dans certaines formes d'épilepsie.

La dose usuelle variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les composés de formule (V) ou (VIII) utilisés au départ peuvent être préparés selon un procédé analogue à celui décrit dans la demande de brevet européen 90.403526.8 selon le schéma réactionnel suivant :

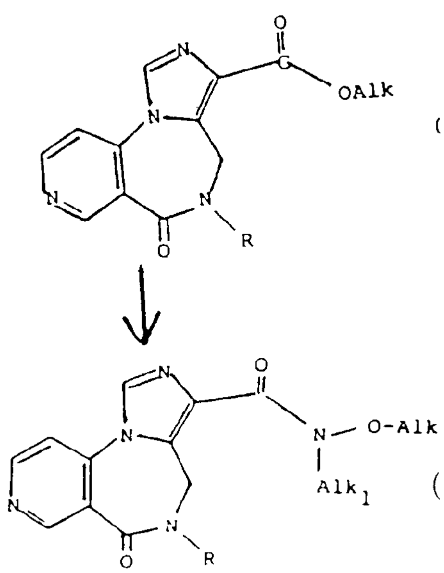

(A)

(V)

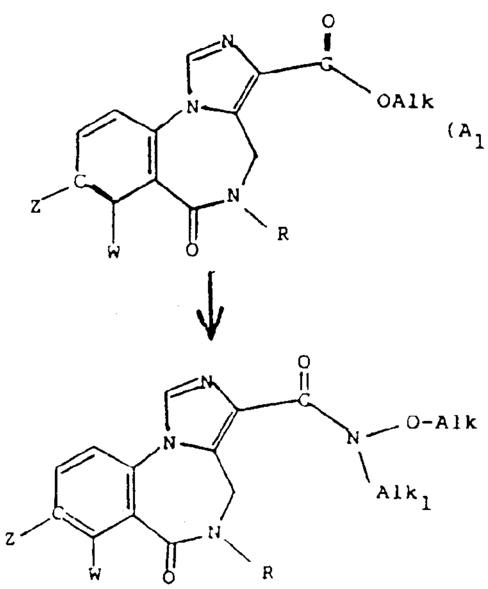

($A_1$)

(IX)

Les composés de formule (II) ou (X) peuvent être préparés selon le procédé indiqué dans le brevet européen n° 27214 ou selon des procédés analogues.

Les produits intermédiaires de formule (VII) :

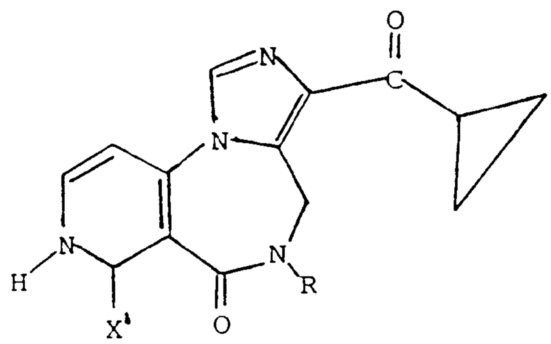

(VII)

dans laquelle X′ et R ont la signification indiquée ci-dessus, de formule (V) :

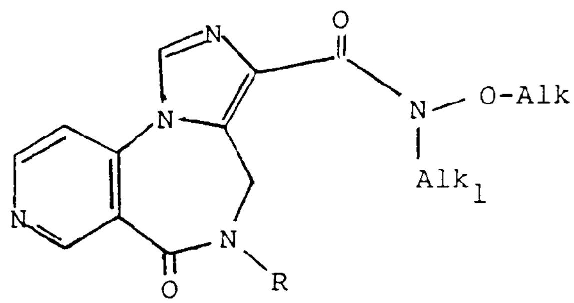

(V)

dans laquelle R, Alk et $Alk_1$ ont la signification indiquée ci-dessus, et de formule (VIII) :

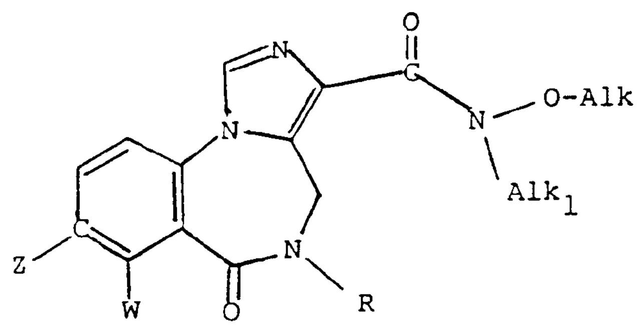

(VIII)

dans laquelle Z, W, R, Alk et $Alk_1$ ont la signification indiquée ci-dessus, sont des produits nouveaux et l'invention a donc aussi pour objet à titre de produits industriels nouveaux les produits de formule (V), (VII) et (VIII) tels que définis ci-dessus.

Il va être donné maintenant à titre non limitatif, des exemples de mise en oeuvre de l'invention.

**Exemple 1 : 7-cyclopropyl-5,6-dihydro-5-méthyl-6-oxo 4H-imidazo [1,5-a] [1,4] benzodiazépin-3-yl cyclopropyl méthanone**

Dans une solution de bromure de cyclopropyl magnésium préparée à partir de 905 mg de magnésium et de 1,63 g de bromure de cyclopropyle dans 25 $cm^3$ de tétrahydrofuranne à 0°C, on ajoute 3,4 g de 7-fluoro 5,6-dihydro 5-méthyl 6-oxo 4H-imidazo [1,5-a] [1,4] benzodiazépin-3-yl N-méthyl carbohydroxamate de méthyle. On agite le mélange pendant 30 minutes puis le verse dans une solution de chlorure d'ammonium et extrait à l'acétate d'éthyle. On sèche et évapore le solvant, chromatographie le résidu sur silice (éluant : chlorure de méthylène-acétate d'éthyle 1-1) et obtient 680 mg de produit attendu. F = 204-205°C.

Spectre IR :

3095, 1634, 1562, 1490, 1360, 1253, 974, 935 $cm^{-1}$

Spectre RMN ($CDCl_3$) :

7,89 (1H,s) ; 7,46 (1H,t) ; 7,17 (1H,d) ; 7,06 (1H,d) ; 5,25 (1H,d) ; 4,32 (1H,d) ; 3,18 (4H,s+m); 2,39 (1H,m) ; 0,95-1,3 (6H,m) ; 0,84 (1H,m) ; 0,67 (1H,m).

Analyse : $C_{19}H_{19}N_3O_2$

| | | | |
|---|---|---|---|
| Calculé : | C% 71,01 | H% 5,96 | N% 13,08 |
| Trouvé : | 70,76 | 6,05 | 12,9 |

**Exemple 2 : 7-cyclopropyl-5,6-dihydro-5-méthyl-6-oxo 4H-imidazo [1,5-a] pyrido [3,4-f] diazépin-3-yl cyclopropyl méthanone**

Stade A : 7-cyclopropyl-5,6,7,8-tétrahydro-5-méthyl-6-oxo 4H-imidazo [1,5-a] pyrido [3,4-f] diazépin-3-yl cyclopropyl méthanone

A 305 mg de (5,6-dihydro 5-méthyl 6-oxo 4H-imidazo [1,5-a] pyrido [3,4-f] diazépin-3-yl) N-méthyl carbohydroxamate de méthyle dans 10 $cm^3$ de tétrahydrofuranne à température ambiante, on ajoute une solution de bromure de cyclopropyl magnésium préparée à partir de 72 mg de magnésium et de 450 mg de bromure de cyclopropyle dans 10 $cm^3$ de tétrahydrofuranne et agite le mélange pendant 1 heure à température ambiante. On ajoute 10 $cm^3$ de chlorure d'ammonium, dilue avec du chlorure de méthylène et de l'eau. On sèche la solution organique et évapore le solvant. Après chromatographie du résidu sur silice (éluant : acétate d'éthyle-chlorure de méthylène 1-1), on obtient 104 mg de produit attendu.

Stade B : 7-cyclopropyl-5,6-dihydro-5-méthyl-6-oxo 4H-imidazo [1,5-a] pyrido [3,4-f] diazépin-3-yl cyclopropyl méthanone

A 78 mg de produit obtenu au stade précédent en solution dans 6 $cm^3$ de chlorure de méthylène, on ajoute 400 mg de dioxyde de manganèse et agite le mélange pendant 1 heure et demie à température ambiante. On filtre et évapore le solvant. Après chromatographie sur silice (éluant : chlorure de méthylène-éther-méthanol 50-50-1), on obtient 61 mg de produit attendu. F = 235-237°C.

Spectre IR :

1643, 1566, 1550, 1490, 1420, 1386, 1203, 1042, 967 $cm^{-1}$

Spectre RMN ($CDCl_3$) :

8,57 (1H,d) ; 7,93 (1H,s) ; 7,04 (1H,d) ; 5,32 (1H,d) ; 4,38 (1H,d) ; 3,17 (4H,s+m); 2,51 (1H,m) ; 1,17 (8H,m).

Analyse : $C_{18}H_{18}N_4O_2$

| | | | |
|---|---|---|---|
| Calculé : | C% 67,07 | H% 5,63 | N% 17,38 |
| Trouvé : | 66,6 | 5,65 | 17,21 |

**Exemple 3 : 5,6-dihydro 5-méthyl 6-oxo 4H-imidazo [1,5-a] pyrido [3,4-f] diazépin-3-yl cyclopropyl méthanone.**

Stade A : 5,6-dihydro 5-méthyl 6-oxo 4H-imidazo [1,5-a] pyrido [3,4-f] diazépin-3-yl cyclopropyl carbinol.

Dans une solution de bromure de cyclopropyl magnésium préparée à partir de 72 mg de magnésium et de 362 mg de bromure de cyclopropyle dans 4 $cm^3$ de tétrahydrofuranne, on ajoute à température ambiante, 240 mg de 5,6-dihydro 5-méthyl 6-oxo 4H-imidazo [1,5-a] pyrido [3,4-f] [1,4] diazépin 3-carboxaldéhyde en suspension dans 1 $cm^3$ de tétrahydrofuranne et 1 $cm^3$ d'hexaméthyl phosphotriamide. On agite 1 heure, verse le milieu réactionnel dans une solution aqueuse de chlorure d'ammonium et extrait au chlorure de méthylène. Après chromatographie sur silice (éluant : chlorure de méthylène-méthanol 95-5), on obtient 120 mg de produit

attendu.

Stade B : 5,6-dihydro 5-méthyl 6-oxo 4H-imidazo [1,5-a] pyrido [3,4-f] diazépin-3-yl cyclopropyl méthanone.

A 100 mg de produit obtenu au stade précédent en solution dans 15 $cm^3$ de chlorure de méthylène, on ajoute 600 mg de dioxyde de manganèse et chauffe le mélange au reflux pendant 40 minutes. On refroidit, filtre et évapore le solvant. On cristallise par addition d'éther et obtient 84 mg de produit attendu. F= 237-238°C.

Spectre IR :

1645, 1564, 1489, 1383, 1170, 1010, 964 $cm^{-1}$

Spectre RMN ($CDCl_3$) :

9,28(1H,s); 8,85(1H,d); 7,97(1H,s); 7,36(1H,d); 4,88(2H,bs); 3,26(3H,s); 3,18(1M,m); 1,25(2H,m); 1,12(2H,m).

Analyse : $C_{15}H_{14}N_4O_2$, 1/2 $H_2O$

| | | | | | | |
|---|---|---|---|---|---|---|
| Calculé : | C% | 62,82 | H% | 5,10 | N% | 19,53 |
| Trouvé : | | 62,97 | | 5,12 | | 19,43 |

**Exemple 4 :**

On a préparé des comprimés répondant à la formulation suivante :

- Produit de l'exemple 1 ...................... 20 mg
- Excipient q.s.p. un comprimé terminé à ...... 150 mg

(détail de l'excipient : lactose, amidon, talc, stéarate de magnésium).

**Exemple 5 :**

On a préparé des comprimés répondant à la formulation suivante :

- Produit de l'exemple 2 ...................... 20 mg
- Excipient q.s.p. un comprimé terminé à ...... 150 mg

(détail de l'excipient : lactose, amidon, talc, stéarate de magnésium).

**ACTIVITE PHARMACOLOGIQUE**

Test N°1

L'affinité des composés pour les récepteurs des benzodiazépines a été évaluée en utilisant un radioligand [$^3$H] flunitrazépam et la méthode de Squires et Braestrup (Nature, 1977, 266, 732 modifiée).

Les valeurs indiquées dans le tableau ci-après sont les concentrations nanomolaires (mol x $10^{-9}$) du produit testé qui inhibent dans une proportion de 50% la liaison spécifique de 0,6 nanomoles de [$^3$H] flunitrazépam dans des préparations de membranes de cerveaux antérieurs de rats [($CI_{50}$) en nanomoles].

Test N°2

L'évaluation de la liaison aux récepteurs des benzodiazépines, in vivo, a été effectuée selon la méthode décrite par Goeders N.E. et Kuhar M.J., (Life Sciences (1985) 37, 345.

TABLEAU II

| Exemple | Test 1 nM | Test 2 DE 50 mg/kg IP |
|---|---|---|
| 1 | 36 | 0,15 |
| 2 | 26 | 0,08 |
| 3 | 105 | 0,13 |

**Revendications**

**1)** Nouvelles 7-Cycloalkyl-imidazodiazépines répondant à la formule générale (I) :

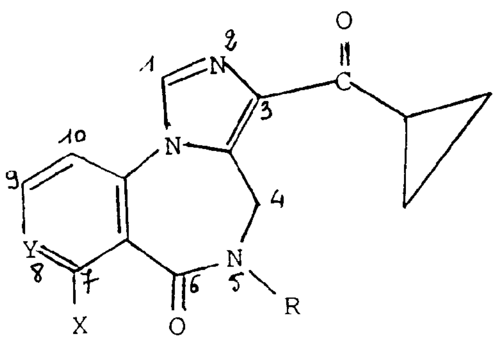

(I)

dans laquelle :

– Y représente un atome d'azote, un atome de carbone lié à un substituant Z dans lequel Z représente un atome d'hydrogène, un atome d'halogène, un groupement nitrile, un groupement

– $N_3$, un groupement -$C(Hal)_3$ dans lequel Hal représente un atome d'halogène,

– X représente un groupement cycloalkyle renfermant de 3 à 6 atomes de carbone ou un atome d'hydrogène étant entendu que lorsque X représente un atome d'hydrogène, Y représente un atome d'azote,

– R représente un atome d'hydrogène, un radical alkyle renfermant de 1 à 5 atomes de carbone, un groupement phényle, un groupement phényle substitué, ainsi que leurs sels d'addition avec les acides minéraux ou organiques.

**2)** Nouvelles 7-Cycloalkyl-imidazodiazépines telles que définies par la formule (I) de la revendication 1, caractérisées en ce que dans ladite formule (I), Y représente un atome d'azote ou un atome de carbone lié à un substituant Z dans lequel Z représente un atome d'hydrogène, X et R ont la signification indiquée à la revendication 1, ainsi que leurs sels d'addition avec les acides minéraux ou organiques.

**3)** Nouvelles 7-Cycloalkyl-imidazodiazépines répondant à la formule (I), selon la revendication 1 ou 2, caractérisées en ce que dans ladite formule (I), X représente un groupement cyclopropyle, Y et R ont la signification indiquée à la revendication 1, ainsi que leurs sels d'addition avec les acides minéraux ou organiques.

**4)** La 7-cyclopropyl-5,6-dihydro-5-méthyl-6-oxo 4H-imidazo [1,5-a] [1,4] benzodiazépin-3-yl cyclopropyl méthanone ainsi que ses sels d'addition avec les acides minéraux ou organiques.

**5)** La 7-cyclopropyl-5,6-dihydro-5-méthyl-6-oxo 4H-imidazo [1,5-a] pyrido [3,4-f] diazépin-3-yl cyclopropyl méthanone ainsi que ses sels d'addition avec les acides minéraux ou organiques.

**6)** Procédé de préparation des nouvelles 7-Cycloalkyl-imidazodiazépines telles que définies par la formule (I) de la revendication 1, ainsi que de leurs sels, caractérisé en ce

a) pour préparer des produits de formule (I) dans laquelle Y représente un atome d'azote,

soit l'on soumet un composé de formule (II) :

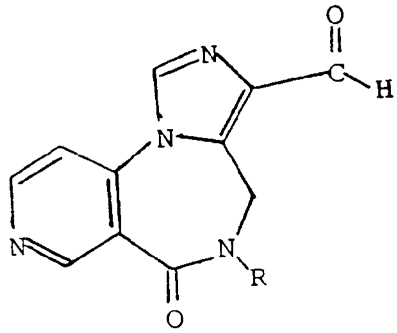

(II)

dans laquelle R a la signification déjà indiquée à l'action d'un dérivé magnésien d'un composé de formule (III) :

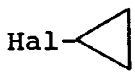

(III)

dans laquelle Hal représente un atome d'halogène pour obtenir un composé de formule (IV) :

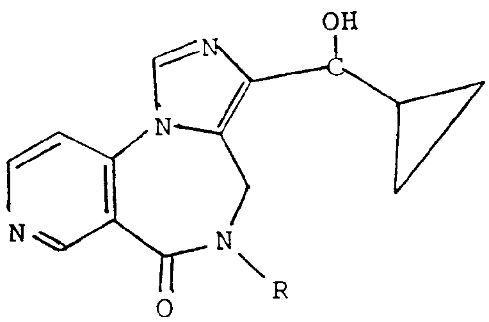

(IV)

dans laquelle R a la signification déjà indiquée, que l'on soumet à l'action d'oxydation pour obtenir le composé de formule (I) correspondant dans laquells X représente un atome d'hydrogène répondant à la formule ($I_A$) :

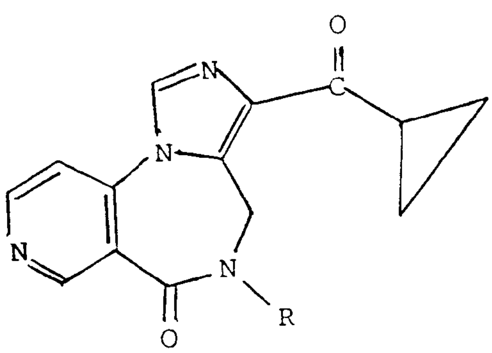

($I_A$)

dans laquelle R a la signification indiquée précédemment, que l'on peut salifier, si désiré,
soit l'on soumet un produit de formule (V) :

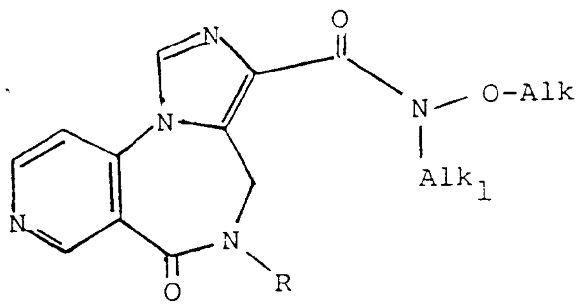

(V)

dans laquelle R a la signification indiquée précédemment et alk et $alk_1$ identiques ou différents représentent un radical alkyle renfermant de 1 à 3 atomes de carbone, à l'action d'un dérivé magnésien d'un composé de formule (III) :

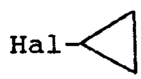

(III)

tel que défini ci-dessus
pour obtenir un produit de formule ($I_A$) tel que défini ci-dessus, que l'on peut salifier si désiré,
produit de formule ($I_A$) que l'on soumet le cas échéant à l'action d'un dérivé magnésien d'un composé de formule (VI) :

Hal-X' (VI)

dans laquelle Hal représente un atome d'halogène et X' représente un radical cycloalkyle renfermant de 3 à 6 atomes de carbone pour obtenir un composé de formule (VII) :

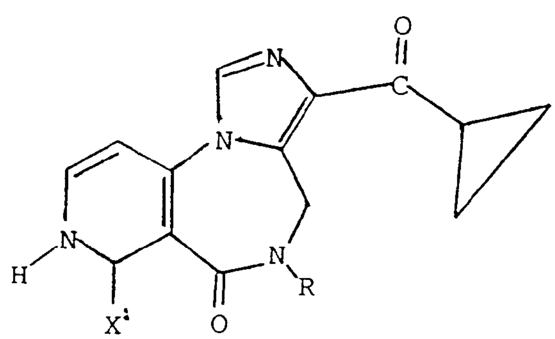

(VII)

dans laquelle X' et R ont la signification indiquée précédemment que l'on soumet à l'action d'un agent d'oxydation pour obtenir le composé de formule (I) correspondant dans laquelle X représente un radical cycloalkyle renfermant de 3 à 6 atomes de carbone répondant à la formule ($I_B$) :

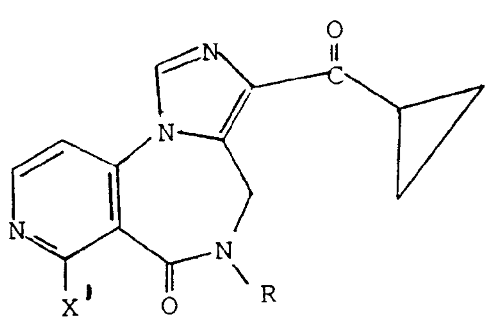

($I_B$)

dans laquelle X' et R ont la signification indiquée précédemment, que l'on peut salifier si désiré,
b) pour préparer des produits de formule (I) dans laquelle Y représente un atome de carbone lié à un substituant Z tel que défini précédemment,
soit l'on soumet un composé de formule (VIII) :

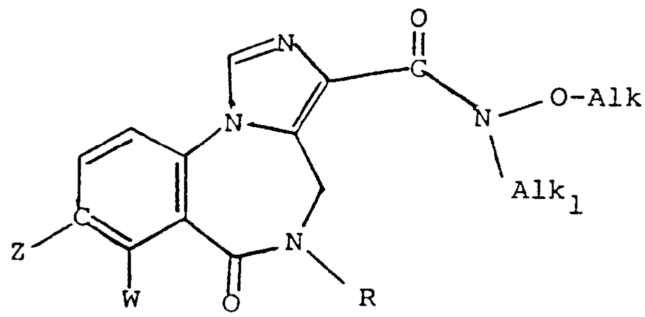

(VIII)

dans laquelle R, Z, Alk et $Alk_1$ ont la signification indiquée précédemment et W représente un atome de fluor ou de chlore, à l'action d'un dérivé magnésien d'un composé de formule (III) :

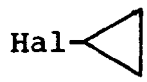

(III)

tel que défini précédemment pour obtenir un composé de formule (IX) :

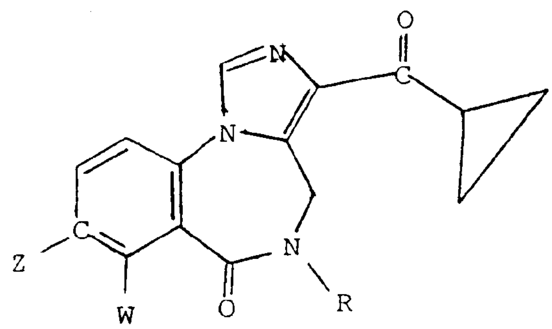

(IX)

dans laquelle Z, W et R ont la signification indiquée précédemment,
soit l'on soumet un composé de formule (X) :

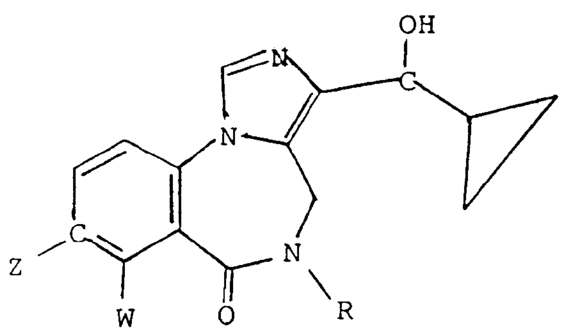

(X)

dans laquelle Z, W et R ont la signification indiquée précédemment, à l'action d'un dérivé magnésien d'un composé de formule (III) :

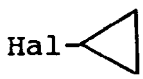

(III)

tel que défini ci-dessus pour obtenir un composé de formule (XI) :

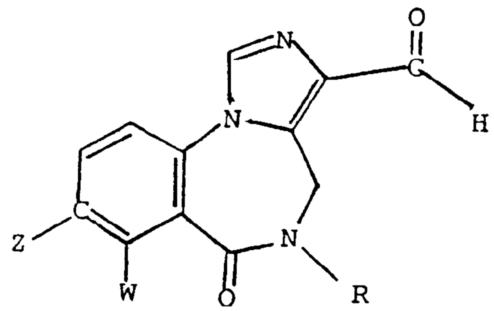

(XI)

dans laquelle Z, W et R ont la signification indiquée précédemment, que l'on soumet à l'action d'un agent d'oxydation pour obtenir un composé de formule (IX) tel que défini précédemment, composé de formule (IX) que l'on soumet à l'action d'un dérivé magnésien d'un composé de formule (VI) :

Hal-X′ (VI)

tel que défini ci-dessus pour obtenir le composé de formule (I) correspondant dans laquelle X représente un radical cycloalkyle répondant à la formule ($I_C$) :

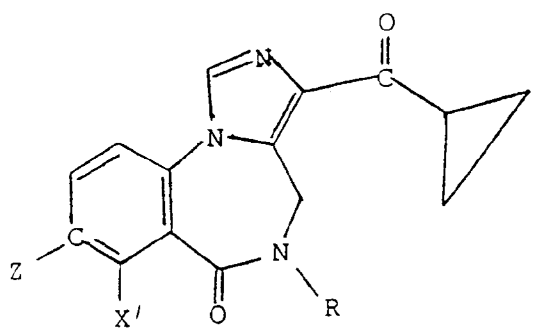

($I_C$)

dans laquelle Z, X′ et R ont la signification indiquée précédemment que l'on peut salifier, si désiré.

**7)** Procédé selon la revendication 6, caractérisé en ce que :

– la réaction du produit de formule (II), (V), (VIII) ou (X) avec le dérivé magnésien d'un produit de formule (III) ou la réaction du produit de formule ($I_A$) ou (IX) avec le dérivé magnésien d'un produit de formule (VI) est effectuée au sein d'un solvant organique anhydre tel que le tétrahydrofuranne,

– l'oxydation du produit de formule (IV), (VII) ou (XI) est réalisée au moyen d'un oxydant doux tel que le dioxyde de manganèse, au sein d'un solvant organique anhydre tel que le dichlorométhane,

– dans le composé de formule (VIII) ou (X), W représente un atome de fluor,

– les composés de formule ($I_B$) ou ($I_C$) dans laquelle X′ représente un radical cyclopropyle sont préparés en traitant respectivement le composé de formule (V) ou (VIII) telle que définie ci-dessus avec un excès du dérivé magnésien du composé de formule (III) tel que défini ci-dessus sans isoler intermédiairement les composés ($I_A$) et (IX) respectivement.

**8)** Médicaments, caractérisés en ce qu'ils sont constitués par les nouvelles 7-cycloalkyl-imidazodiazépines telles que définies par la formule (I) de la revendication 1, ainsi que par leurs sels pharmaceutiquement acceptables.

**9)** Médicaments, caractérisés en ce qu'ils sont constitués par les nouvelles 7-cycloalkyl-imidazodiazépines telles que définies à l'une quelconque des revendications 2 à 5, ainsi que par leur sels pharmaceutiquement acceptables.

**10)** Compositions pharmaceutiques, caractérisées en ce qu'elles renferment à titre de principe actif, l'un au moins des médicaments tels que définis à l'une quelconque des revendications 8 ou 9.

**11)** A titre de produits industriels nouveaux, les produits de formule :

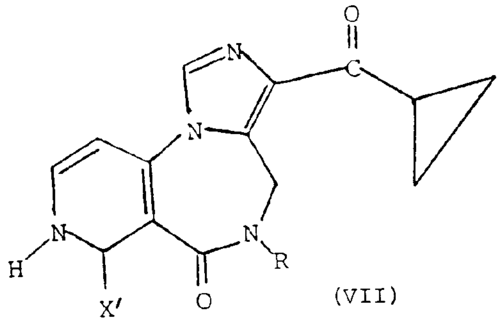

(VII)

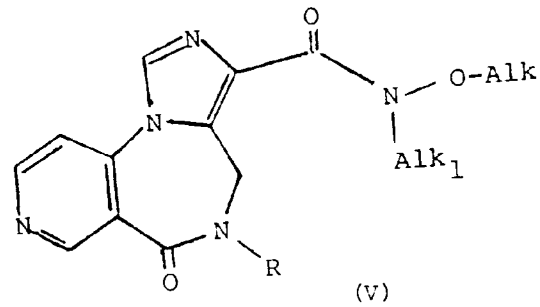

(V)

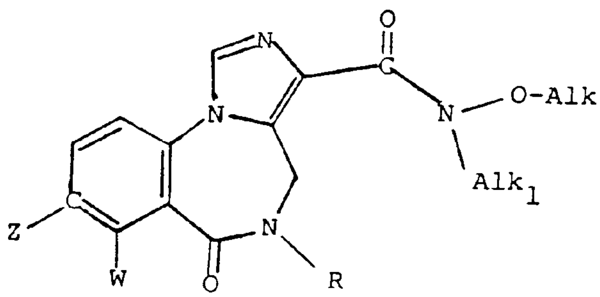

(VIII)

dans lesquelles X′, R, Alk, $Alk_1$, W et Z ont la signification indiquée à la revendication 1.

**Revendications pour l'Etat contractant suivant: ES.**

**1.-** Procédé pour préparer des nouvelles 7-cycloalkylimidazodiazépines répondant à la formule générale (I) :

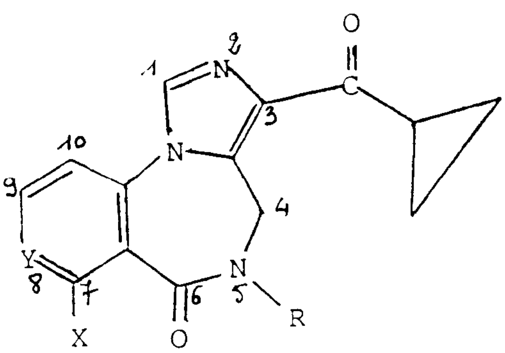

(I)

dans laquelle :

– Y représente un atome d'azote, un atome de carbone lié à un substituant Z dans lequel Z représente un atome d'hydrogène, un atome d'halogène, un groupement nitrile, un groupement $-N_3$, un groupement $-C(Hal)_3$ dans lequel Hal représente un atome d'halogène,

– X représente un groupement cycloalkyle renfermant de 3 à 6 atomes de carbone ou un atome d'hydrogène étant entendu que lorsque X représente d'hydrogène, Y représente un atome d'azote,

– R représente un atome d'hydrogène, un radical alkyle renfermant de 1 à 5 atomes de carbone, un groupement phényle, un groupement phényle substitué, ainsi que de leurs sels d'addition avec les acides minéraux ou organiques, caractérisé en ce que :

a) pour préparer des produits de formule (I) dans laquelle Y représente un atome d'azote,

soit l'on soumet un composé de formule (II) :

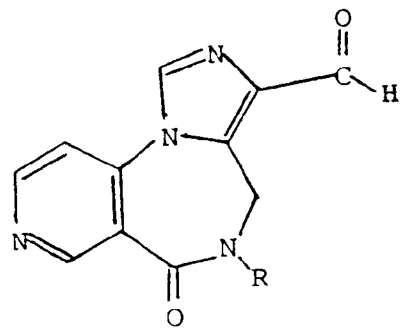

(II)

dans laquelle R a la signification déjà indiquée à l'action d'un dérivé magnésien d'un composé de formule (III) :

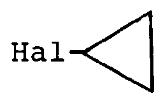

dans laquelle Hal représente un atome d'halogène, pour obtenir un composé de formule (IV) :

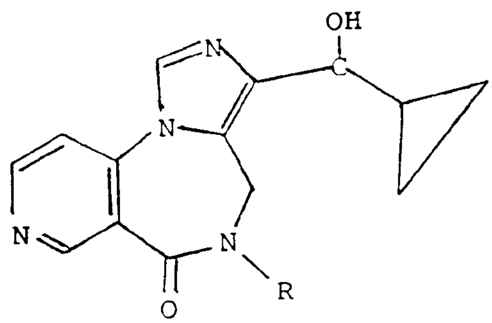

(IV)

dans laquelle R a la signification déjà indiquée, que l'on soumet à l'action d'oxydation pour obtenir le composé de formule (I) correspondant dans laquelle X représente un atome d'hydrogène répondant à la formule ($I_A$) :

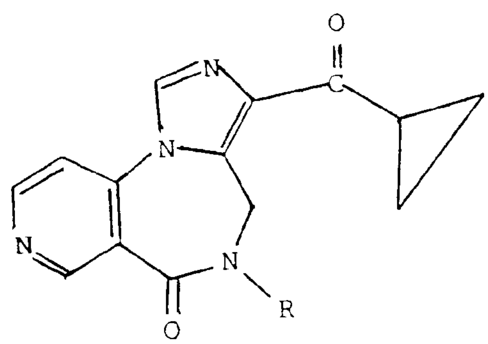

($I_A$)

dans laquelle R a la signification indiquée précédemment, que l'on peut salifier, si désiré,
soit l'on soumet un produit de formule (V) :

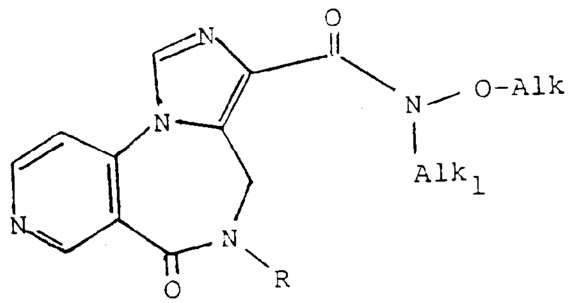

(V)

dans laquelle R a la signification indiquée précédemment et alk et $alk_1$ identiques ou différents représentent un radical alkyle renfermant de 1 à 3 atomes de carbone, à l'action d'un dérivé magnésien d'un composé de formule (III) :

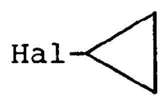

tel que défini ci-dessus,
pour obtenir un produit de formule ($I_A$) tel que défini ci-dessus, que l'on peut salifier si désiré,
produit de formule ($I_A$) que l'on soumet le cas échéant à l'action d'un dérivé magnésien d'un composé de formule (VI) :

Hal-X′ (VI)

dans laquelle Hal représente un atome d'halogène et X′ représente un radical cycloalkyle renfermant de 3 à 6 atomes de carbone pour obtenir un composé de formule (VII) :

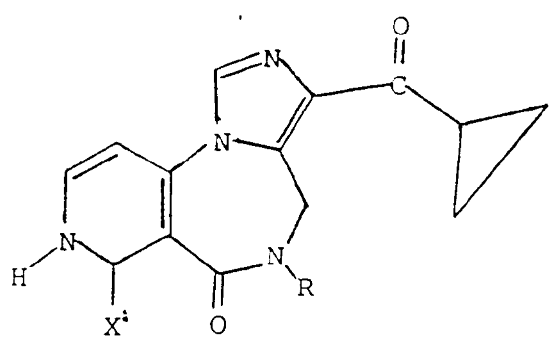

(VII)

dans laquelle X′ et R ont la signification indiquée précédemment que l'on soumet à l'action d'un agent d'oxydation pour obtenir le composé de formule (I) correspondant dans laquelle X représente un radical cycloalkyle renfermant de 3 à 6 atomes de carbone répondant à la formule ($I_B$) :

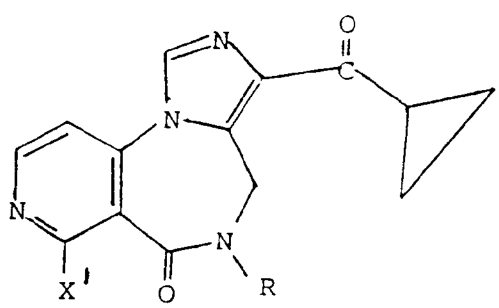

($I_B$)

dans laquelle X′ et R ont la signification indiquée précédemment, que l'on peut salifier si désiré,
b) pour préparer des produits de formule (I) dans laquelle Y représente un atome de carbone lié à un substituant Z tel que défini précédemment,

soit l'on soumet un composé de formule (VIII) :

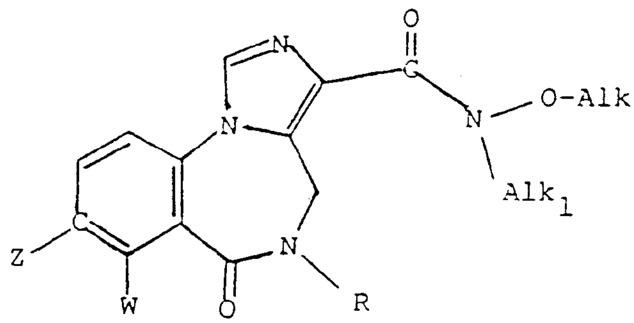

(VIII)

dans laquelle R, Z, Alk et $Alk_1$ ont la signification indiquée précédemment et W représente un atome de fluor ou de chlore, à l'action d'un dérivé magnésien d'un composé de formule (III) :

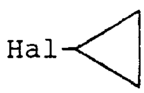

(III)

et tel que défini précédemment pour obtenir un composé de formule (IX) :

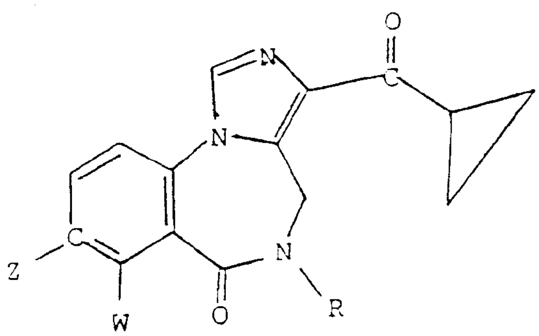

(IX)

dans laquelle Z, W et R ont la signification indiquée précédemment,
soit l'on soumet un composé de formule (X) :

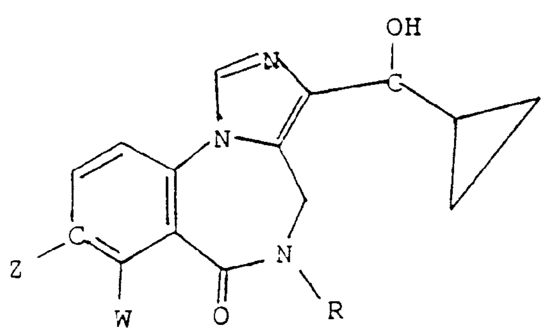

(X)

dans laquelle Z, W et R ont la signification indiquée précédemment, à l'action d'un dérivé magnésien d'un composé de formule (III) :

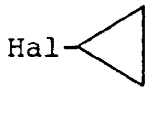

(III)

tel que défini ci-dessus pour obtenir un composé de formule (XI) :

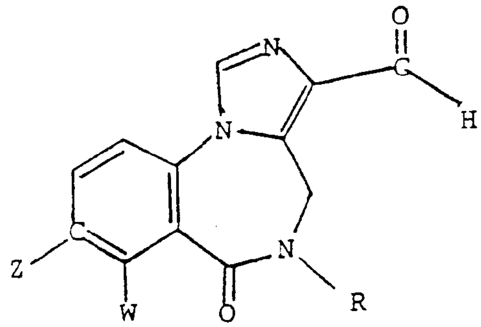

(XI)

dans laquelle Z, W et R ont la signification indiquée précédemment, que l'on soumet à l'action d'un agent d'oxydation pour obtenir un composé de formule (IX) tel que défini précédemment, composé de formule (IX) que l'on soumet à l'action d'un dérivé magnésien d'un composé de formule (VI) :

Hal-X' (VI)

tel que défini ci-dessus pour obtenir le composé de formule (I) correspondant dans laquelle X représente un radical cycloalkyle répondant à la formule ($I_C$) :

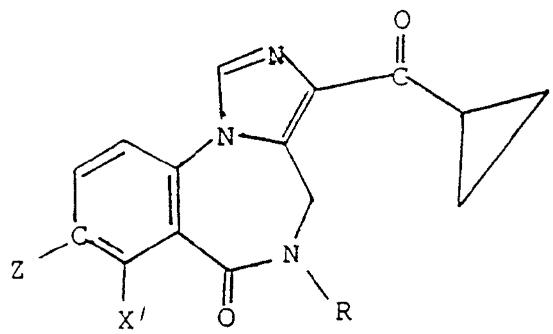

($I_C$)

dans laquelle Z, X' et R ont la signification indiquée précédemment que l'on peut salifier, si désiré.

**2.-** Procédé selon la revendication 1, caractérisé en ce que :

– la réaction du produit de formule (II), (V), (VIII) ou (X) avec le dérivé magnésien d'un produit de formule (III) ou la réaction du produit de formule ($I_A$) ou (IX) avec le dérivé magnésien d'un produit de formule (VI) est effectuée au sein d'un solvant organique anhydre tel que le tétrahydrofuranne,

– l'oxydation du produit de formule (IV), (VII) ou (XI) est réalisée au moyen d'un oxydant doux tel que le dioxyde de manganèse, au sein d'un solvant organique anhydre tel que le dichlorométhane,

Dans le composé de formule (VIII) ou (X), W représente avantageusement un atome de fluor.

– Les composés de formule ($I_B$) ou ($I_C$) dans laquelle X' représente un radical cyclopropyle sont préparés en traitant respectivement le composé de formule (V) ou (VIII) telle que définie ci-dessus avec un excès du dérivé magnésien du composé de formule (III) tel que défini ci-dessus sans isoler intermédiairement les composés de formule ($I_A$) ou (IX) respectivement.

**3.-** Procédé selon la revendication 1, caractérisé en ce que l'on utilise au départ un produit de formule (II) ou (V).

**4.-** Procédé selon la revendication 1, caractérisé en ce que l'on utilise au départ un produit de formule (VIII) ou (X) dans laquelle Z représente un atome d'hydrogène.

**5.-** Procédé selon l'une quelconque des revendications 1 à 4, caractérisé en ce que l'on utilise au départ un produit de formule (VI) dans laquelle X' représente un radical cyclopropyle.

**Revendications pour l'Etat contractant suivant: GR.**

**1.-** Procédé pour préparer des nouvelles 7-cycloalkylimidazodiazépines répondant à la formule générale (I) :

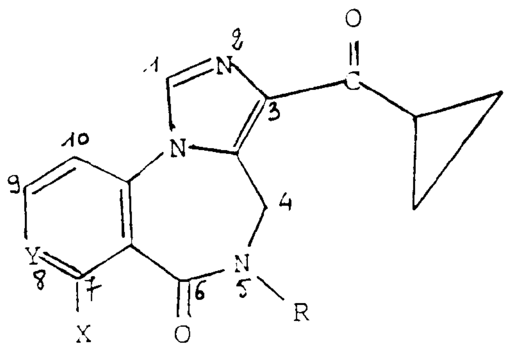

(I)

dans laquelle :

– Y représente un atome d'azote, un atome de carbone lié à un substituant Z dans lequel Z représente un atome d'hydrogène, un atome d'halogène, un groupement nitrile, un groupement $-N_3$, un groupement $-C(Hal)_3$ dans lequel Hal représente un atome d'halogène,

– X représente un groupement cycloalkyle renfermant de 3 à 6 atomes de carbone ou un atome d'hydrogène étant entendu que lorsque X représente d'hydrogène, Y représente un atome d'azote,

– R représente un atome d'hydrogène, un radical alkyle renfermant de 1 à 5 atomes de carbone, un groupement phényle, un groupement phényle substitué, ainsi que de leurs sels d'addition avec les acides minéraux ou organiques, caractérisé en ce que :

a) pour préparer des produits de formule (I) dans laquelle Y représente un atome d'azote, soit l'on soumet un composé de formule (II) :

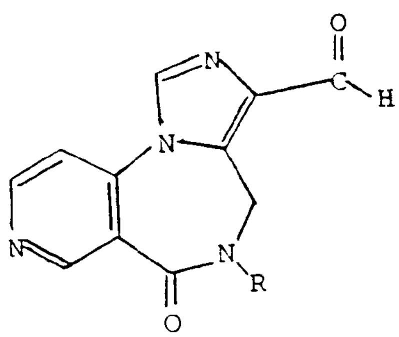

(II)

dans laquelle R a la signification déjà indiquée à l'action d'un dérivé magnésien d'un composé de formule (III) :

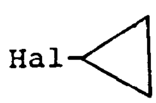

dans laquelle Hal représente un atome d'halogène, pour obtenir un composé de formule (IV) :

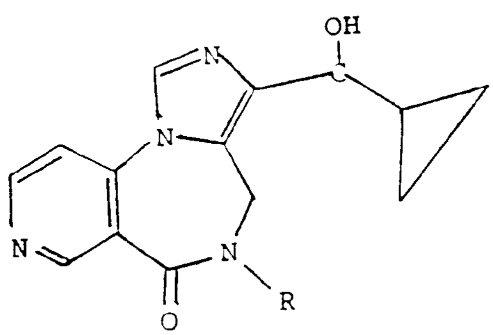

(IV)

dans laquelle R a la signification déjà indiquée, que l'on soumet à l'action d'oxydation pour obtenir le composé de formule (I) correspondant dans laquelle X représente un atome d'hydrogène répondant à la formule ($I_A$) :

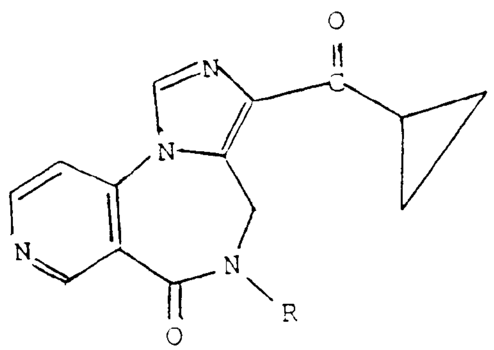

($I_A$)

dans laquelle R a la signification indiquée précédemment, que l'on peut salifier, si désiré,
soit l'on soumet un produit de formule (V) :

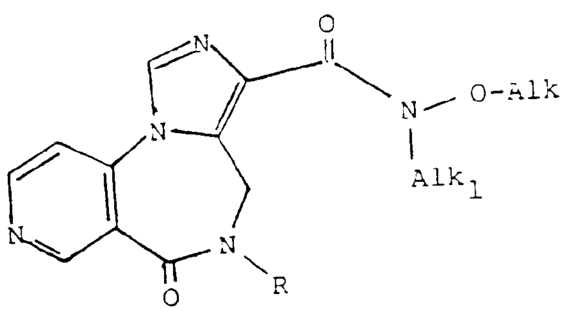

(V)

dans laquelle R a la signification indiquée précédemment et alk et $alk_1$ identiques ou différents représentent un radical alkyle renfermant de 1 à 3 atomes de carbone, à l'action d'un dérivé magnésien d'un composé de formule (III) :

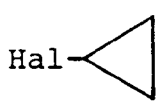

tel que défini ci-dessus,
pour obtenir un produit de formule ($I_A$) tel que défini ci-dessus, que l'on peut salifier si désiré,
produit de formule ($I_A$) que l'on soumet le cas échéant à l'action d'un dérivé magnésien d'un composé de formule (VI) :

Hal-X' (VI)

dans laquelle Hal représente un atome d'halogène et X' représente un radical cycloalkyle renfermant de 3 à 6 atomes de carbone pour obtenir un composé de formule (VII) :

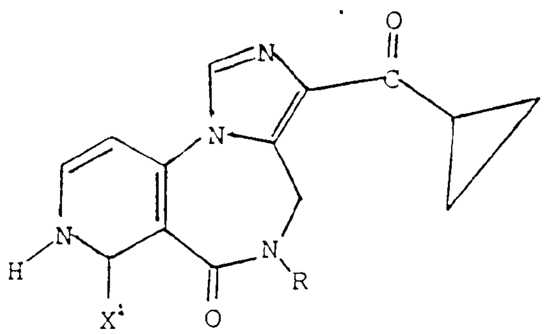

(VII)

dans laquelle X' et R ont la signification indiquée précédemment que l'on soumet à l'action d'un agent d'oxydation pour obtenir le composé de formule (I) correspondant dans laquelle X représente un radical cycloalkyle renfermant de 3 à 6 atomes de carbone répondant à la formule ($I_B$) :

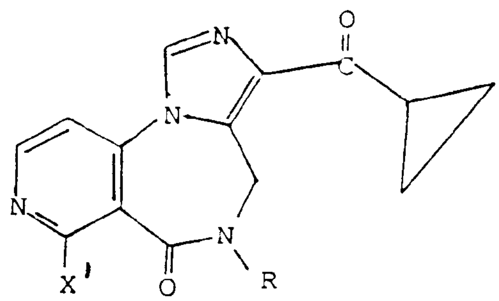

($I_B$)

dans laquelle X' et R ont la signification indiquée précédemment, que l'on peut salifier si désiré,
b) pour préparer des produits de formule (I) dans laquelle Y représente un atome de carbone lié à un substituant Z tel que défini précédemment,
soit l'on soumet un composé de formule (VIII) :

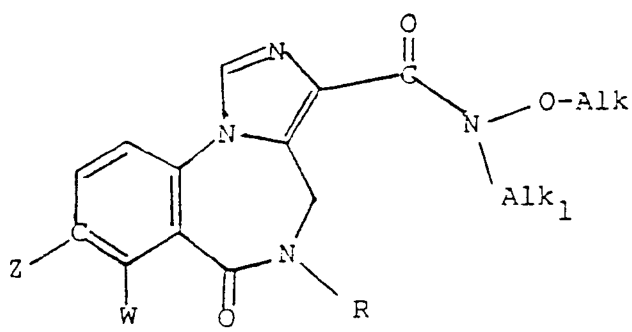

(VIII)

dans laquelle R, Z, Alk et $Alk_1$ ont la signification indiquée précédemment et W représente un atome de fluor ou de chlore, à l'action d'un dérivé magnésien d'un composé de formule (III) :

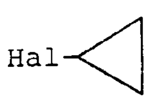

(III)

et tel que défini précédemment pour obtenir un composé de formule (IX) :

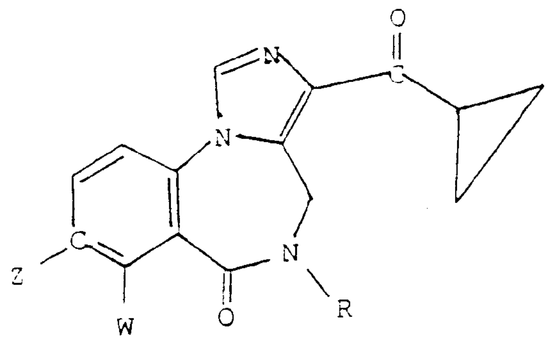

(IX)

dans laquelle Z, W et R ont la signification indiquée précédemment, soit l'on soumet un composé de formule (X) :

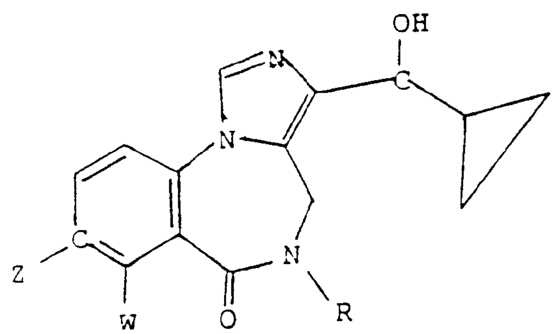

(X)

dans laquelle Z, W et R ont la signification indiquée précédemment, à l'action d'un dérivé magnésien d'un composé de formule (III) :

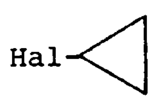

(III)

tel que défini ci-dessus pour obtenir un composé de formule (XI) :

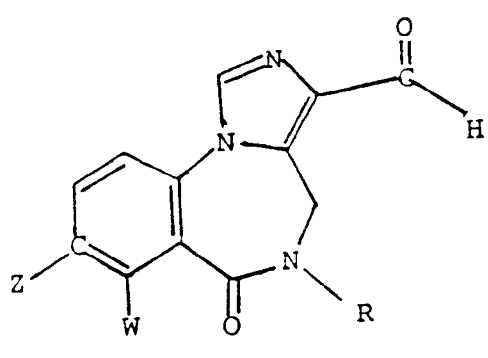

(XI)

dans laquelle Z, W et R ont la signification indiquée précédemment, que l'on soumet à l'action d'un agent d'oxydation pour obtenir un composé de formule (IX) tel que défini précédemment, composé de formule (IX) que l'on soumet à l'action d'un dérivé magnésien d'un composé de formule (VI) :

Hal-X' (VI)

tel que défini ci-dessus pour obtenir le composé de formule (I) correspondant dans laquelle X représente un radical cycloalkyle répondant à la formule ($I_c$) :

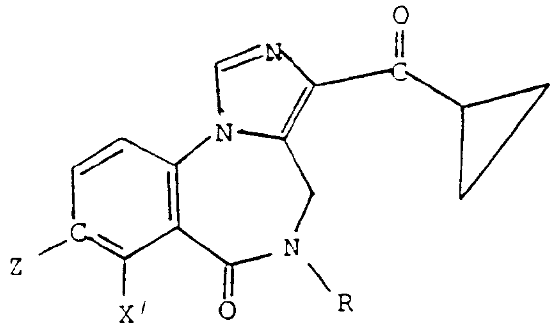

($I_C$)

dans laquelle Z, X′ et R ont la signification indiquée précédemment que l'on peut salifier, si désiré.

**2.-** Procédé selon la revendication 1, caractérisé en ce que :

– la réaction du produit de formule (II), (V), (VIII) ou (X) avec le dérivé magnésien d'un produit de formule (III) ou la réaction du produit de formule ($I_A$) ou (IX) avec le dérivé magnésien d'un produit de formule (VI) est effectuée au sein d'un solvant organique anhydre tel que le tétrahydrofuranne,

– l'oxydation du produit de formule (IV), (VII) ou (XI) est réalisée au moyen d'un oxydant doux tel que le dioxyde de manganèse, au sein d'un solvant organique anhydre tel que le dichlorométhane,

Dans le composé de formule (VIII) ou (X), W représente avantageusement un atome de fluor.

– Les composés de formule ($I_B$) ou ($I_C$) dans laquelle X′ représente un radical cyclopropyle sont préparés en traitant respectivement le composé de formule (V) ou (VIII) telle que définie ci-dessus avec un excès du dérivé magnésien du composé de formule (III) tel que défini ci-dessus sans isoler intermédiairement les composés de formule ($I_A$) ou (IX) respectivement.

**3.-** Procédé selon la revendication 1, caractérisé en ce que l'on utilise au départ un produit de formule (II) ou (V).

**4.-** Procédé selon la revendication 1, caractérisé en ce que l'on utilise au départ un produit de formule (VIII) ou (X) dans laquelle Z représente un atome d'hydrogène.

**5.-** Procédé selon l'une quelconque des revendications 1 à 4, caractérisé en ce que l'on utilise au départ un produit de formule (VI) dans laquelle X′ représente un radical cyclopropyle.

**6.-** Procédé selon la revendication 1, 2 ou 4, caractérisé en ce que l'on choisit au départ un produit de formule (VIII) de manière telle que l'on prépare la 7-cyclopropyl-5,6-dihydro-5-méthyl-6-oxo 4H-imidazo [1,5-a] [1,4] benzodiazépin-3-yl cyclopropyl méthanone.

**7.-** Procédé selon la revendication 1, 2 ou 3, caractérisé en ce que l'on choisit au départ un produit de formule (V) de manière telle que l'on prépare la 7-cyclopropyl-5,6-dihydro-5-méthyl-6-oxo 4H-imidazo [1,5-a] pyrido [3,4-f] diazépin-3-yl cyclopropyl méthanone.

**8.-** Procédé pour préparer des compositions pharmaceutiques, caractérisé en ce que l'on met à titre de principe actif l'un au moins des produits de formule (I) telle que définie à la revendication 1 ou l'un au moins de leurs sels pharmaceutiquement acceptables, sous une forme destinée à cet usage.

**9.-** Procédé pour préparer des compositions pharmaceutiques, caractérisé en ce que l'on met à titre de principe actif l'un au moins des produits de formule (I) telle que définie à l'une quelconque des revendications 2 à 7 ou l'un au moins de leurs sels pharmaceutiquement acceptables, sous une forme destinée à cet usage.

**10.-** A titre de produits industriels nouveaux les produits de formule

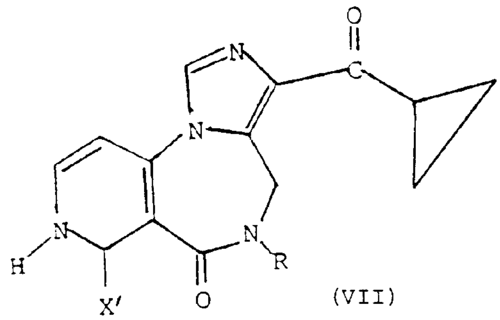

(VII)

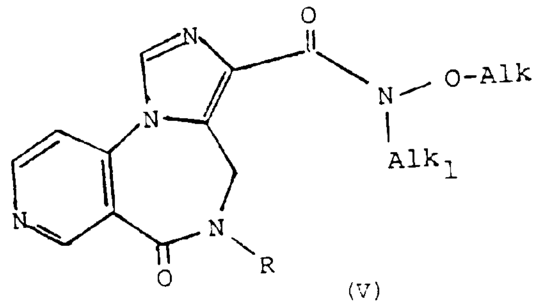

(V)

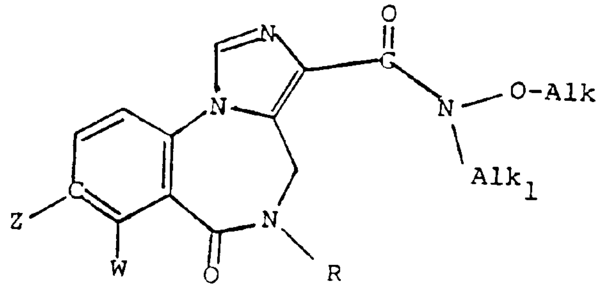

(VIII)

dans lesquelles X', R, alk, $alk_1$, W et Z ont la signification indiquée à la revendication 1.

Office européen des brevets

# RAPPORT DE RECHERCHE EUROPEENNE

Numero de la demande

EP 91 40 2825

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 305 298 (ROUSSEL-UCLAF)<br>* revendication 12 * | 11 | C07D487/04<br>C07D471/14 |
| A | * page 7, ligne 43 - ligne 46; revendication 1 * | 1,8 | A61K31/55<br>//(C07D487/04, |
| A | EP-A-0 059 388 (HOFFMANN-LA ROCHE)<br>* page 17, ligne 35 - page 18, ligne 5; revendication 1 * | 1,8 | 243:00,235:00)<br>(C07D471/14,<br>243:00,221:00) |
| A | GB-A-2 174 695 (MERCK SHARP AND DOHME)<br>* page 3, ligne 14 - ligne 16; revendication 1 * | 1,8 | |
| P,X | EP-A-0 433 163 (ROUSSEL-UCLAF)<br>* revendication 16 * | 11 | |
| P,A | * page 9, ligne 16 - ligne 33; revendications 1,11 * | 1,8 | |
| | | | **DOMAINES TECHNIQUES RECHERCHES (Int. Cl.5)** |
| | | | C07D |

Le présent rapport a été établi pour toutes les revendications

| Lieu de la recherche | Date d'achèvement de la recherche | Examinateur |
|---|---|---|
| LA HAYE | 17 FEVRIER 1992 | HEYWOOD C.J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)